(19) Europäisches Patentamt
European Patent Office
Office européen des brevets

(11) **EP 3 602 000 B1**

(12) **FASCICULE DE BREVET EUROPEEN**

(45) Date de publication et mention de la délivrance du brevet:
**28.12.2022 Bulletin 2022/52**

(21) Numéro de dépôt: **18715211.1**

(22) Date de dépôt: **20.03.2018**

(51) Classification Internationale des Brevets (IPC):
**G01N 3/40** *(2006.01)*   **G01N 3/42** *(2006.01)*
**C12M 1/42** *(2006.01)*

(52) Classification Coopérative des Brevets (CPC):
**G01N 3/40; C12M 35/06; G01N 3/42;**
G01N 2203/0078; G01N 2203/0085;
G01N 2203/0087; G01N 2203/0089;
G01N 2203/0286

(86) Numéro de dépôt international:
**PCT/FR2018/050670**

(87) Numéro de publication internationale:
**WO 2018/172688 (27.09.2018 Gazette 2018/39)**

(54) **DISPOSITIF POUR LA CARACTERISATION MECANIQUE D'UN ELEMENT D'INTERET PAR EXEMPLE UN OVOCYTE**

VORRICHTUNG ZUR MECHANISCHEN CHARAKTERISIERUNG EINES BESTIMMTEN ELEMENTES WIE OOZYTEN

DEVICE FOR MECHANICALLY CHARACTERIZING AN ELEMENT OF INTEREST SUCH AS AN OOCYTE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.03.2017 FR 1752330**

(43) Date de publication de la demande:
**05.02.2020 Bulletin 2020/06**

(73) Titulaires:
• **Centre National de la Recherche Scientifique**
**75016 Paris (FR)**
• **Universite De Franche Comte**
**25000 Besançon (FR)**
• **Ecole Nationale Superieure de Mecanique et Des Microtechniques**
**25000 Besancon (FR)**

(72) Inventeurs:
• **ABADIE, Joel**
**25000 Besancon (FR)**
• **GANA, Racha**
**Monastir 5000 (TN)**
• **PIAT, Emmanuel**
**25660 Fontain (FR)**

(74) Mandataire: **Jacobacci Coralis Harle**
**32, rue de l'Arcade**
**75008 Paris (FR)**

(56) Documents cités:
WO-A1-2017/020006     WO-A2-2008/105919
US-A- 5 486 457     US-A- 5 723 793
US-A1- 2009 068 701     US-A1- 2011 053 241
US-A1- 2014 312 251

• L. GUILLOU ET AL: "dynamic monitoring of cell mechanical properties using profile microindentation", SCIENTIFIC REPORTS, vol. 6, 9 février 2016 (2016-02-09), pages 1-13, XP002775574,

**Description**

DOMAINE TECHNIQUE AUQUEL SE RAPPORTE L'INVENTION

La présente invention concerne, de manière générale, le domaine de la caractérisation mécanique d'un élément d'intérêt.

Elle concerne plus particulièrement un dispositif adapté à la caractérisation mécanique d'un élément d'intérêt microscopique, éventuellement de nature biologique, voire une cellule, de préférence un ovocyte.

ARRIERE-PLAN TECHNOLOGIQUE

La caractérisation mécanique d'un élément d'intérêt consiste à analyser ses propriétés intrinsèques (module d'Young, coefficient de Poisson, etc.), à partir de tests et d'essais mécaniques.

La plupart des essais actuels sont destinés à caractériser des métaux et des polymères à l'échelle macroscopique.

Ils ne sont généralement pas adaptés pour caractériser les éléments de nature organique, et le sont encore moins à l'échelle microscopique.

Or, la caractérisation mécanique des cellules vivantes constitue une problématique à part entière, notamment dans le domaine de l'assistance médicale à la procréation.

En effet, une telle caractérisation mécanique est probablement un critère important à prendre en compte lors de la sélection d'ovocytes, notamment ceux ayant la capacité à être fécondé.

De nouvelles techniques ont ainsi été développées spécialement pour la caractérisation mécanique des cellules vivantes. Ces techniques consistent généralement à déformer la cellule en lui appliquant une charge ou une contrainte connue, et à mesurer la déformation qui en résulte.

Parmi les techniques les plus employées, il existe la micro-indentation et l'aspiration à l'aide d'une pipette.

Par exemple, le document WO-2008/105919 décrit une méthode pour la caractérisation des propriétés nanomécaniques de cellules, qui utilise pour cela un microscope à force atomique (AFM).

Le document US-2011/053241 décrit un dispositif pour appliquer une

déformation mécanique à des cellules, qui comprend en particulier un micro-actionneur porté par un substrat, apte à être manœuvré entre deux positions par le biais d'un champ magnétique.

Un document Gouillou et al., SCIENTIFIC REPORTS, vol. 6, 2016-02-09, décrit une méthode pour suivre les propriétés mécaniques d'une cellule, par l'utilisation d'un profil de microindentation. Le microindenteur mis en œuvre comprend pour cela une tige (tip), destinée à être orientée verticalement dont une extrémité supérieure est portée par un contrôleur piezoélectrique, pour assurer sa manœuvre en translation.

Mais, en pratique, ces techniques actuelles offrent des possibilités de caractérisation très limitées.

Il existe par conséquent un besoin pour un dispositif qui permettrait une caractérisation mécanique par indentation pour mesurer les efforts appliqués sur un élément d'intérêt microscopique (notamment une cellule vivante), de manière performante, fiable et étalonnée.

OBJET DE L'INVENTION

Dans ce contexte, la présente invention propose un dispositif pour la caractérisation mécanique d'un élément d'intérêt, avantageusement un élément d'intérêt microscopique, éventuellement de nature biologique, voire une cellule, de préférence un ovocyte.

Plus particulièrement, on propose selon l'invention un dispositif de caractérisation mécanique tel que défini dans la revendication 1 et ses dépendantes.

En pratique, les moyens magnétiques permettent d'appliquer une charge connue à l'élément d'intérêt, par l'intermédiaire de l'organe indenteur.

Pour cela, cet organe indenteur est maintenu dans un champ magnétique destiné à varier de manière contrôlée.

Le champ magnétique mis en œuvre présente alors les propriétés :

- il préserve une direction horizontale instable pour l'organe indenteur,
- il assure le maintient stable de l'organe indenteur, hors direction de mesure, et
- il agit comme un ressort magnétique à raideur négative (ou le cas échéant à raideur positive) sur l'organe indenteur dans ladite direction horizontale instable, pour effectuer le chargement sur l'élément d'intérêt.

La raideur du ressort magnétique est négative (ou le cas échéant positive) dans la direction horizontale instable ; l'élément d'intérêt assure alors la stabilité de l'organe indenteur qui le comprime.

Le système est donc stabilisé par l'élément d'intérêt que l'on souhaite caractériser, ce qui confère une simplicité et une efficacité optimales.

Avec cette technologie, il est possible de mettre en œuvre des raideurs de l'ordre de $10^{-3}$ N/m, permettant ainsi de contrôler le chargement à moins de 10 nN près, tout en assurant une excellente linéarité de mesure de la force appliquée.

Un tel dispositif de caractérisation mécanique est en plus intéressant pour son faible coût, sa robustesse et sa facilité de mise en œuvre.

D'autres caractéristiques non limitatives et avantageuses du dispositif de caractérisation mécanique conforme à l'invention, prises individuellement ou selon toutes les combinaisons techniquement possibles, sont les suivantes :

- l'organe indenteur comporte au moins deux aimants permanents qui sont agencés, d'une part, avec des champs magnétiques coaxiaux et dans un même sens NS - NS et, d'autre part, dans une position d'équilibrage horizontal, dans laquelle le centre de poussée est destiné à être confondu avec le centre de gravité ; avantageusement, la poussée d'Archimède produite par le milieu liquide sur l'organe indenteur est égale, et opposée, au poids de l'organe indenteur ;
- ledit organe indenteur présente les caractéristiques suivantes : une longueur comprise entre 1 cm et 3 cm, un diamètre compris entre 0,5 et 1,5 mm, et une masse comprise entre 1 mg et 15 mg ;
- ledit organe indenteur comprend un corps réalisé par un capillaire, par exemple en verre, délimitant une chambre étanche qui est remplie d'air et qui renferme ledit au moins un aimant permanent ;
- le contenant comprend un fond raccordé à une paroi latérale dont une bordure supérieure libre délimite une ouverture supérieure, par exemple une boîte de Pétri ;
- les moyens magnétiques comprennent au moins deux aimants permanents qui sont agencés coaxialement, selon un axe horizontal, et dans le même sens NS - NS, et des moyens pour la manœuvre en translation desdits aimants permanents ; de préférence, lesdits au moins deux aimants permanents sont disposés chacun au sein d'une bobine électromagnétique, lesquelles bobines électromagnétiques sont agencées coaxialement selon l'axe horizontal et sont raccordées à des moyens pour piloter le courant électrique alimentant lesdites bobines électromagnétiques ; lesdits au moins deux aimants permanents, et le cas échéant lesdites au moins deux bobines électromagnétiques, sont avantageusement disposés de part et d'autre du contenant, à une distance constante d l'un par rapport à l'autre ; par exemple, les moyens de manœuvre consistent en des platines de microtranslation ;
- les moyens de maintien comprennent une pipette d'aspiration ;
- les moyens pour déterminer les caractéristiques mécaniques dudit élément d'intérêt comprennent des moyens pour déterminer la valeur de déplacement en translation de l'organe indenteur selon ladite direction horizontale instable ; ces moyens de détermination comprennent avantageusement des moyens optiques, adaptés à la capture d'images comprenant l'élément d'intérêt coopérant avec l'extrémité proximale de l'organe indenteur, et des moyens d'analyse desdites images capturées, adaptés à déterminer la valeur du déplacement en translation dudit organe indenteur selon ladite direction horizontale instable ;
- les moyens supports comportent des moyens pour le chauffage dudit contenant.

L'invention propose également un poste d'injection en procréation médicale assistée, équipé d'un dispositif selon l'invention.

L'invention concerne également un procédé pour étudier les caractéristiques mécaniques d'un élément d'intérêt, avantageusement un élément d'intérêt microscopique, éventuellement de nature biologique, voire une cellule, de préférence un ovocyte, tel que défini dans la revendication 11 et ses dépendantes.

Ce procédé comprend :

a) une phase de préparation au cours de laquelle, d'autre part, ledit élément d'intérêt est maintenu dans le milieu liquide du contenant par lesdits moyens de maintien et, d'autre part, les moyens magnétiques génèrent un champ magnétique initial qui permet le maintien dudit organe indenteur dans une position initiale au repos, à distance dudit élément d'intérêt,

b) une phase de chargement au cours de laquelle les moyens magnétiques sont pilotés de sorte à modifier (éventuellement progressivement) le champ magnétique depuis ledit champ magnétique initial jusqu'à un champ magnétique modifié (avantageusement constant ou évolutif ; par exemple par une manœuvre en translation des moyens magnétiques et/ou par un pilotage du courant électrique injecté dans les bobines électromagnétiques) pour manœuvrer ledit organe indenteur en translation selon ladite direction horizontale instable, cela dans un sens de chargement dans lequel ladite extrémité proximale dudit organe indenteur génère une force de compression sur ledit élément d'intérêt maintenu par lesdits moyens de maintien,

c) une phase de déchargement au cours de laquelle les moyens magnétiques sont pilotés de sorte à rétablir (avantageusement progressivement) ledit champ magnétique initial dans lequel ledit organe indenteur est manœuvré en translation selon ladite direction horizontale instable, cela dans un sens de déchargement dans lequel ladite extrémité proximale dudit organe indenteur s'écarte par rapport audit élément d'intérêt maintenu par lesdits moyens de maintien,

lequel procédé comprend une étape de collecte de la valeur du déplacement en translation dudit organe indenteur selon ladite direction horizontale instable, au moins au cours de la phase de chargement, et lequel procédé comprend une étape de détermination des caractéristiques mécaniques dudit élément d'intérêt, tenant compte de la valeur du déplacement en translation dudit organe indenteur selon ladite direction horizontale instable et des caractéristiques dudit champ magnétique (à savoir, le cas échéant, de la valeur du courant électrique injecté dans les bobines électromagnétiques et/ou de la valeur de déplacement des moyens magnétiques).

De préférence, les étapes de chargement et de déchargement sont effectuées à une très faible vitesse comprise entre 0,1 et 50 micromètres par seconde.

DESCRIPTION DETAILLEE D'UN EXEMPLE DE RÉALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 est une vue générale, et schématique, d'une partie du dispositif de caractérisation mécanique selon l'invention ;
- la figure 2 représente les principales étapes du procédé pour étudier les caractéristiques mécaniques d'un élément d'intérêt, avantageusement un ovocyte, par la mise en œuvre du dispositif de caractérisation mécanique selon la figure 1, avec en particulier une phase de préparation (figure 2A) et une phase de chargement (figure 2B) ;
- la figure 3 est une vue générale, schématique et en perspective, du dispositif de caractérisation mécanique selon les figures 1 et 2 (les moyens supports et les moyens de maintien ne sont pas représentés sur cette figure 3 dans un souci de simplification) ;
- la figure 4 représente un exemple de réponse mécanique d'un ovocyte, mesurée au cours du procédé selon la figure 2 (ordonnée : force en Newton ; abscisse : valeur du déplacement en translation de l'organe indenteur en phase de chargement puis en phase de déchargement).

**Dispositif de caractérisation mécanique**

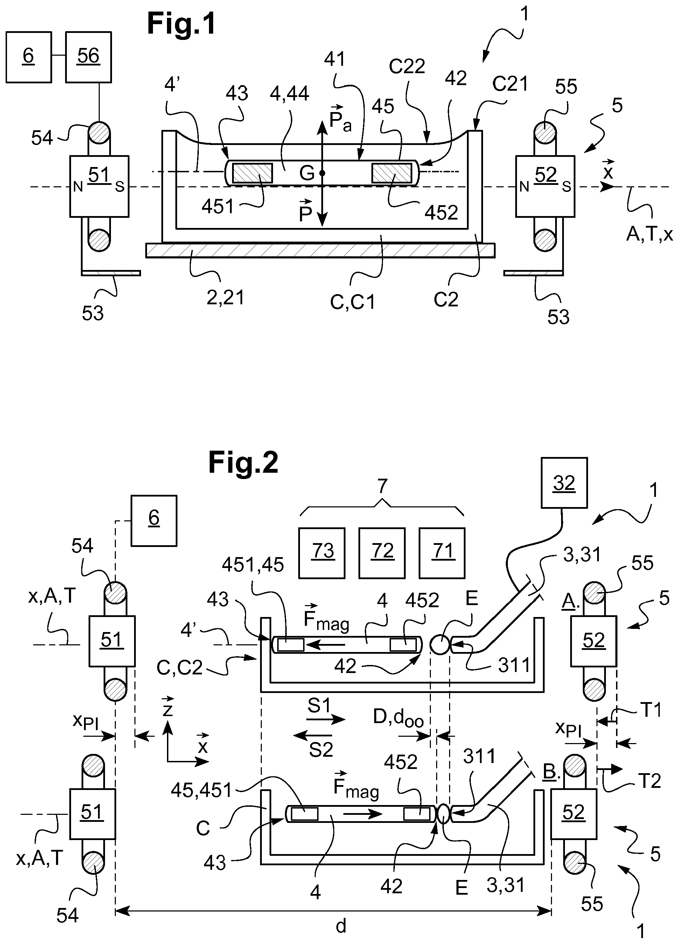

Le dispositif 1 selon l'invention, représenté sur les figures 1 et 2, consiste en un dispositif 1 pour la caractérisation mécanique d'un élément d'intérêt.

Par « caractérisation mécanique », on entend l'analyse des propriétés mécaniques intrinsèques de l'élément d'intérêt, cela à partir de tests et d'essais mécaniques.

Le dispositif de caractérisation mécanique 1 selon l'invention est destiné en particulier à permettre la détermination de la réponse mécanique de l'élément d'intérêt.

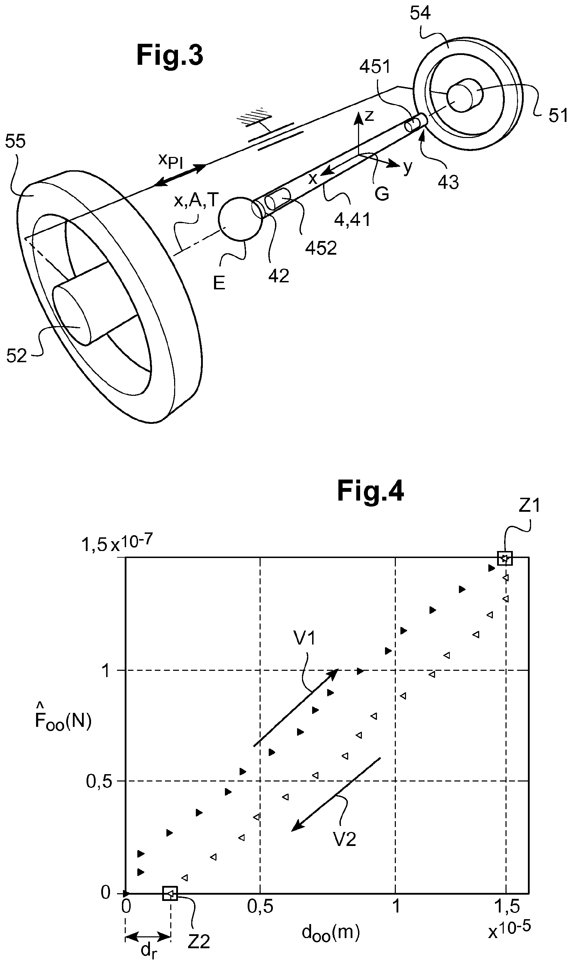

Il permet en particulier d'établir l'évolution de la distance de compression D (par exemple en $\mu$m) en fonction de la force de compression (indentation) $\hat{F}_E$ (par exemple en nN), cela au cours d'une procédure d'indentation (chargement / déchargement) sur cet élément d'intérêt (comme illustré par l'Exemple décrit ci-après en relation avec la figure 4).

Pour cela, selon l'invention, le dispositif de caractérisation mécanique 1 présente un capteur magnétique de nanoforce, qui repose sur l'utilisation d'un ressort magnétique passif et sur l'utilisation de la direction instable de ce ressort magnétique passif pour effectuer les mesures de caractérisation mécanique.

Par « ressort magnétique », on entend un dispositif comprenant un aimant maintenu en équilibre stable par des forces de rappel issues d'action à distance et pour lequel aucun frottement sec venant perturber le mouvement de l'aimant n'est présent. Ces actions sont équivalentes aux actions produites par un ressort non matérialisé dont les extrémités sont reliées, d'une part, à cet aimant et, d'autre part, à un point fixe de référence.

Un ressort magnétique dit «passif» assure la stabilité du dispositif d'indentation, sans apport d'énergie ni asservissement.

Dans ce dispositif de caractérisation mécanique 1, la mesure est effectuée dans une direction horizontale instable x magnétique et en utilisant la réaction répulsive de l'élément d'intérêt E pour stabiliser la direction de mesure.

Pour cela, tel que représenté sur les figures 1 et 2, le dispositif de caractérisation mécanique 1 comprend :

- des moyens supports 2, pour la réception d'un contenant C adapté à contenir un milieu liquide,
- des moyens de maintien 3, pour le maintien de l'élément d'intérêt E dans le milieu liquide,
- un organe indenteur 4 (transducteur du capteur), présentant un axe longitudinal 4' et destiné à rester en sustentation dans le milieu liquide avec ledit axe longitudinal 4' orienté horizontalement,
- des moyens magnétiques 5, pour générer un champ magnétique dans lequel ledit organe indenteur 4 est destiné à se déplacer, qui participent à la sustentation dudit organe indenteur 4 et qui définissent une direction horizontale instable x (matérialisée par l'axe de référence $\vec{x}$ sur les figures 1 à 3),
- des moyens de commande 6, pour commander la manœuvre de l'organe indenteur 4 en translation selon la direction horizontale instable, et
- des moyens de détermination 7, pour déterminer les caractéristiques mécaniques de l'élément d'intérêt E.

De manière générale, dans un souci de simplification, la direction horizontale instable et l'axe de référence précités sont parfois désignés par un même repère $\vec{x}$.

Moyens supports

Les moyens supports 2 sont classiques en soi, par exemple sous la forme d'une plaque.

Ces moyens supports 2 comportent avantageusement des moyens 21 pour le chauffage du contenant C, par exemple sous la forme d'une thermoplaque.

De son côté, le contenant C comprend un fond C1 raccordé à une paroi latérale C2 dont une bordure supérieure libre C21 délimite une ouverture supérieure C22.

Un tel contenant C consiste ainsi avantageusement en une boîte de Pétri, réalisée en verre ou en plastique.

Le milieu liquide consiste quant à lui avantageusement en un milieu aqueux, adapté à l'élément d'intérêt.

Par exemple, dans le cas d'un ovocyte, le milieu aqueux consiste avantageusement en un milieu de culture embryonnaire qui est classique en soi.

Moyens de maintien

Tel que représenté sur la figure 2, les moyens de maintien 3 sont adaptés à maintenir l'élément d'intérêt E dans le milieu liquide (non représenté).

Ces moyens de maintien 3 comprennent ici une pipette d'aspiration 31, dont une extrémité libre 311 est destinée à plonger dans le milieu liquide.

Cette extrémité libre 311 définit avantageusement une surface verticale destinée à s'étendre perpendiculairement à la direction horizontale instable $\vec{x}$.

La pipette d'aspiration 31 est encore associée à des moyens d'aspiration 32, adaptés à assurer la force de préhension de l'élément d'intérêt E au niveau de son extrémité libre 311.

Organe indenteur

L'organe indenteur 4 est destiné, d'une part, à rester en sustentation dans le milieu liquide, avec son axe longitudinal 4' orienté horizontalement et, d'autre part, à être soumis à un effort magnétique capable de le déplacer dans la direction horizontale instable $x$ (matérialisée par l'axe horizontal de référence $\vec{x}$ sur les figures 1 à 3).

De préférence, en pratique, l'axe longitudinal 4' de l'organe indenteur 4 est destiné à être orienté coaxialement (ou au moins parallèlement) autour de la direction horizontale instable $x$ et de l'axe horizontal de référence $\vec{x}$.

En l'espèce, l'organe indenteur 4 comprend un corps 41 qui est de forme allongée et cylindrique.

Le corps 41 comporte deux extrémités, situées sur l'axe longitudinal 4', à savoir une extrémité proximale 42 destinée à indenter l'élément d'intérêt E et une extrémité distale 43, opposée.

La forme de l'extrémité proximale 42 est adaptée à l'essai réalisé et à la forme de l'élément d'intérêt E étudié

Ce corps 41 est formé par un capillaire, par exemple en verre, délimitant une chambre étanche 44 remplie d'air. Cette caractéristique participe à la sustentation de l'organe indenteur 4 dans le milieu liquide.

De préférence, la fabrication de l'organe indenteur 4 permet d'avoir une poussée d'Archimède $\vec{Pa}$ égale (ou au moins quasiment égale), et opposée, à son poids $\vec{P}$.

L'organe indenteur 4 reste ainsi piégé à altitude constante, ou au moins approximativement constante, autour de l'axe de référence horizontal $\vec{x}$.

L'organe indenteur 4 comporte encore au moins un aimant permanent 45 (interne), pour participer, d'une part, à la sustentation dans le milieu liquide et, d'autre part, pour maintenir son axe longitudinal 4' coaxial (ou au moins parallèle) à l'axe horizontal de référence $\vec{x}$.

Par « aimant permanent », on entend un corps ferromagnétique qui produit et entretient un champ magnétique sans l'intervention d'un courant électrique. L'aiment permanent consiste par exemple en un aimant en néodyme fer bore. Un tel aimant permanent comporte classiquement des pôles nord (N) et sud (S).

Plus précisément, ledit au moins un aimant permanent 45 permet à l'organe indenteur 4 de se comporter comme un système relié à un ressort magnétique passif.

En l'espèce, le corps 41 de l'organe indenteur 4 renferme deux aimants permanents 451, 452 (par exemple cylindrique) qui sont ménagés au sein de la chambre étanche 44.

Les aimants permanents 451, 452 sont agencés avec des champs magnétiques coaxiaux et dans un même sens NS - NS.

En d'autre termes, les aimants permanents 451, 452 sont ici agencés coaxialement l'un par rapport à l'autre et par rapport à l'axe longitudinal 4' de l'organe indenteur 4. En outre, le pôle sud de l'un des aimants permanents 451, 452 se situe en regard du pôle nord de l'autre des aimants permanents 451, 452.

Les aimants permanents 451, 452 sont également agencés selon une position d'équilibrage horizontal, dans laquelle le centre de poussée est destiné à être confondu (ou au moins quasiment confondu) avec le centre de gravité G de l'organe indenteur 4.

Uniquement à titre indicatif et sans être aucunement limitatif, l'organe indenteur 4 présente les caractéristiques suivantes :

- une longueur (distance entre les deux extrémités 42, 43 de son corps 41) comprise entre 1 cm et 3 cm,
- un diamètre (section perpendiculaire à l'axe longitudinal 4') compris entre 0,5 et 1,5 mm, et
- une masse comprise entre 1 mg et 15 mg.

Moyens magnétiques

Les moyens magnétiques 5, couplés à l'organe indenteur 4, sont conçus pour former un ressort magnétique passif.

Pour cela, les moyens magnétiques 5 génèrent un champ magnétique dans lequel l'organe indenteur 4 est destiné à se déplacer. Ce champ magnétique participe à la sustentation, au guidage et à l'orientation de cet organe indenteur 4 suivant la direction horizontale instable x.

En particulier, les moyens magnétiques 5 sont capables de générer, suivant les directions $\vec{y}$ et $\vec{z}$, de très faibles forces de rappel qui maintiennent en équilibre (en position et en orientation) l'organe indenteur 4 autour de l'axe horizontal de référence $\vec{x}$.

On notera que les directions y et z consistent en des directions perpendiculaires à la direction horizontale instable x, orientées respectivement horizontalement et verticalement.

Ces moyens magnétiques 5 sont en outre destinés à exercer une force magnétique $\vec{F}_{mag}$, contrôlée sur l'organe indenteur 4, orientée coaxialement à la direction horizontale instable x (figure 2).

Cette force magnétique $\vec{F}_{mag}$ conditionne les déplacements et l'équilibre de l'organe indenteur 4 selon la direction horizontale instable x.

En l'espèce, cette force magnétique instable $\vec{F}_{mag}$ correspond à la résultante de la force magnétique ramenée au centre de gravité G de l'organe indenteur 4.

A cet effet, les moyens magnétiques 5 comprennent ici deux aimants permanents 51, 52 (externes), du type cylindrique par exemple, qui sont agencés coaxialement, selon un axe horizontal A et dans le même sens NS - NS.

L'axe horizontal A de ces deux aimants permanents 51, 52 est confondu avec l'axe horizontal de référence $\vec{x}$. En d'autres termes, la magnétisation des aimants permanents 51, 52 est coaxiale à l'axe horizontal A.

L'axe horizontal A de ces deux aimants permanents 51, 52 définit ainsi l'axe horizontal de référence $\vec{x}$ et la direction horizontale instable x.

Les deux aimants permanents 51, 52 sont disposés de part et d'autre du contenant C, à une distance constante d l'un par rapport à l'autre.

Ces deux aimants permanents 51, 52 sont ici disposés chacun au sein d'une bobine électromagnétique 54, 55.

Les deux bobines électromagnétiques 54, 55 sont agencées coaxialement par rapport à l'axe horizontal A précité des deux aimants permanents 51, 52 associés.

Ces deux bobines électromagnétiques 54, 55 sont avantageusement câblées en série et orientées SN - NS.

Ainsi, les deux aimants permanents 51, 52, et les deux bobines électromagnétiques 54, 55, sont disposés de part et d'autre du contenant C, à une distance constante d l'un par rapport à l'autre.

Les moyens magnétiques 5 comprennent également des moyens 53 pour la manœuvre en translation de ces deux aimants permanents 51, 52, et des bobines électromagnétiques 54, 55 associées, selon une direction horizontale T qui est orientée coaxialement à l'axe horizontal A.

Cette direction horizontale T est ainsi coaxiale, ou au moins parallèle, à la direction horizontale instable x.

Cette manœuvre en translation selon la direction horizontale T des deux aimants permanents 51, 52, et des bobines électromagnétiques 54, 55, est destinée à s'effectuer sur une distance $x_{PI}$ contrôlée et déterminée (figure 2).

Par exemple, ces moyens de manœuvre 53 consistent en des platines de microtranslation.

Les moyens magnétiques 5 comprennent encore des moyens 56 pour piloter le courant électrique alimentant les bobines électromagnétiques 54, 55, de sorte à générer une variation du champ magnétique destiné à assurer (ou au moins à participer à) une manœuvre en translation de l'organe indenteur 4 selon la direction horizontale instable x.

Par exemple, ces moyens de pilotage 56 consistent en un circuit d'alimentation électrique, classique en soi, apte à générer un courant électrique d'une valeur déterminée dans les bobines électromagnétiques 54, 55.

## Moyens de commande

Les moyens de commande 6 sont destinés à piloter les moyens magnétiques 5 de sorte à générer une variation du champ magnétique. Cette variation de champ magnétique assure alors une manœuvre en translation de l'organe indenteur 4, selon la direction horizontale instable $\vec{x}$.

Ces moyens de commande 6 consistent avantageusement en une partie commande d'un système automate programmable industriel. Ils comprennent en particulier un programme d'ordinateur comportant des moyens de code de programme destinés à être exécutés par un ordinateur.

Ces moyens de commande 6 sont en particulier conçus pour piloter ici :

- les moyens 53 pour la manœuvre en translation de ces deux aimants permanents 51, 52 et des bobines électromagnétiques 54, 55 associées, et/ou
- les moyens 56 pour piloter le courant électrique appliqué aux bobines électromagnétiques 54, 55.

Comme abordé précédemment, la manœuvre en translation des deux aimants permanents 51, 52 et des bobines électromagnétiques 54, 55 associées, selon la direction horizontale T, s'effectue avantageusement sur une distance $x_{PI}$ contrôlée et déterminée (figure 2).

## Moyens de détermination

Les moyens de détermination 7 sont configurés pour déterminer les caractéristiques mécaniques de l'élément d'intérêt E, cela tenant compte :

- des caractéristiques du champ magnétique généré par les moyens magnétiques 5 (par exemple de la constante de raideur du ressort magnétique) et

- de la valeur D du déplacement en translation de l'organe indenteur 4 selon la direction horizontale instable x, lorsque l'organe indenteur 4 génère une force de compression sur l'élément d'intérêt E (figure 2B).

Les moyens de détermination 7 comprennent des moyens 71, 72 pour déterminer la valeur D de déplacement en translation de l'organe indenteur 4 selon la direction horizontale instable x.

Pour déterminer cette valeur de déplacement D, les moyens de détermination 7 comprennent par exemple des moyens optiques 71, adaptés à la capture d'images comprenant l'élément d'intérêt E coopérant avec l'extrémité proximale 42 de l'organe indenteur 4 (figure 2B).

Ces moyens optiques 71 comprennent par exemple un système de caméra / tube / objectif, placé au-dessus du contenant C.

Ces moyens optiques 71 permettent de suivre les déformations de l'élément d'intérêt E, mais aussi de calculer par traitement d'image automatisé les positions de l'organe indenteur 4.

Encore pour déterminer cette valeur de déplacement D, les moyens de détermination 7 comprennent également avantageusement des moyens d'analyse 72 qui sont adaptés à déterminer, à partir des images capturées par les moyens optiques 71, la valeur D du déplacement en translation de l'organe indenteur 4 selon la direction horizontale instable x.

En d'autres termes, cette valeur de déplacement D correspond à la distance entre, d'une part, une position initiale $x_i^{init}$ de l'organe indenteur 4 et, d'autre part, une position finale $x_i^{max}$ de l'organe indenteur 4 (avantageusement provoqué par le déplacement $x_{PI}$ des aimants permanents 51, 52 et deux bobines électromagnétiques 54, 55 des moyens magnétiques 5 et/ou par le courant injecté dans les deux bobines électromagnétiques 54, 55 associées).

De manière générale, cette valeur D est encore désignée « $d_{oo}$ » dans le cas d'un élément d'intérêt du type ovocyte.

Les moyens d'analyse 72 comprennent encore avantageusement un programme d'ordinateur du type logiciel d'analyse d'image, comportant des moyens de code de programme destinés à être exécutés par un ordinateur.

Ces moyens de code de programme utilisent par exemple un algorithme de traitement d'image, basé sur la méthode de corrélation croisée normalisée (CCN).

En pratique, la charge appliquée à l'élément d'intérêt E est modulée à partir de la mesure de la valeur de déplacement D (dite encore « distance de compression »), du contrôle de la distance $x_{PI}$ de manœuvre en translation des deux aimants permanents 51, 52 et/ou du contrôle du courant injecté dans les deux bobines électromagnétiques 54, 55 associées.

Les moyens de détermination 7 comprennent encore des moyens de calcul 73 (par exemple un programme d'ordinateur) qui sont configurés pour déterminer, partant des données capturées précitées, les caractéristiques mécaniques de l'élément d'intérêt E.

Ces caractéristiques mécaniques comprennent avantageusement une courbe représentant l'évolution de la valeur de déplacement D (compression) en fonction de la valeur de la force $\hat{F}_E$ (effort appliqué par l'élément d'intérêt E sur l'organe indenteur 4), au cours d'une procédure d'indentation (en chargement, voire également en déchargement) sur cet élément d'intérêt E.

Une distance de compression maximale $D^{max}$ (à appliquer à l'élément d'intérêt pour un test donné) peut être fixée (prédéterminée).

La force $\hat{F}_E^{max}$ est la valeur de la force $\hat{F}_E$ qui est obtenue pour :

- un déplacement des aimants permanents 51, 52 externes sur la distance $x_{PI}$ prédéterminée et/ou
- une valeur prédéterminée de courant injecté dans les deux bobines électromagnétiques 54, 55 associées.

La particularité de ce dispositif réside dans le fait que l'instabilité de l'organe indenteur 4 suivant l'axe horizontal de référence x est évitée en créant un effort égal, et opposé, à la force magnétique instable $\vec{F}_{mag}$ en plaçant l'élément d'intérêt E (par exemple l'ovocyte) en face de l'organe indenteur 4.

A l'équilibre, on aura ainsi :

$$\vec{F}_{mag} + \vec{F}_E = 0$$

où $\vec{F}_E$ (dit encore $\vec{F}_{oo}$ pour un ovocyte) est l'effort appliqué par l'élément d'intérêt E sur l'organe indenteur 4.

Le débattement relatif à appliquer entre l'organe indenteur 4 et les aimants permanents 51, 52 externes, et/ou le courant injecté dans les deux bobines électromagnétiques 54, 55 associées, se situeront chacun dans une fourchette déterminée.

Lors d'un chargement idéal, on s'efforce de maintenir la vitesse de l'organe indenteur 4 constante et de faible amplitude.

Dans ces conditions :

$$\hat{F}_E = K\left(x_i - x_i^{init} - x_{PI} + x_{PI}^{init}\right) + K_{elec}I$$

avec

$\hat{F}_E$ la force appliquée à l'élément d'intérêt (en N),

K, la raideur du ressort magnétique (obtenue par exemple à partir de simulations réalisées avec les paramètres réels du dispositif) en N/m,

$x_{PI}$ le déplacement appliqué aux aimants permanents 51, 52 des moyens magnétiques 5,

$x_i$, l'ensemble des positions de l'organe indenteur,

$K_{elec}$, la raideur électrique des bobines électromagnétiques 54, 55 (en N/A),

I, le courant injecté dans les bobines électromagnétiques 54, 55 (en A), ces bobines électromagnétiques 54, 55 étant câblées en série et orientées SN-NS,

$x_{PI}^{init}$ et $x_i^{init}$, les positions initiales des aimants permanents 51, 52 et des bobines électromagnétiques 54, 55 appartenant aux moyens magnétiques 5 et de l'organe indenteur 4, respectivement.

De son côté, la distance de compression $D^{max}$ est la valeur de la distance de compression D, obtenue pour un déplacement de la distance $x_{PI}$ maximale prédéterminée et/ou ou un courant I maximal prédéterminé.

Cette distance D de compression de l'élément d'intérêt E (désignée « $d_{oo}$ » dans le cas d'un élément d'intérêt du type ovocyte) est donnée par la formule suivante :

$$D = U_{\frac{pixel}{m}}\left(x_{px} - x_{px}^{init}\right)$$

dans laquelle :

$U_{pixel}$ est le gain de conversion entre les mesures de déplacement fournies par m le système de vision (en pixel) et les déplacements réels de l'organe indenteur 4 (en m),

$x_{px}$, l'ensemble des positions de l'organe indenteur 4 mesurées en pixel, et

$x_{px}^{init}$, la position initiale de l'organe indenteur 4 mesurée en pixel.

A partir de ces valeurs, les moyens de détermination 7 peuvent tracer une courbe de réponse mécanique de l'élément d'intérêt (voir par exemple la figure 4).

De manière générale, la distance D de compression pourrait être mesurée par le biais de tous moyens appropriés, autres que des moyens optiques.

## Procédé pour étudier les caractéristiques mécaniques d'un élément d'intérêt

La mise en œuvre du dispositif de caractérisation mécanique 1 est décrite ci-dessous en relation avec la figure 2. Ce procédé de mise en œuvre comprend les phases successives suivantes.

Tout d'abord, ce procédé est initié par une phase de préparation (figure 2A), au cours de laquelle l'élément d'intérêt E est positionné et maintenu, dans le milieu liquide du contenant C, par les moyens de maintien 3.

En l'espèce, l'élément d'intérêt E est maintenu par aspiration à l'aide de la pipette de contention 31.

D'une manière générale, l'organe indenteur 4, couplé aux moyens magnétiques 5, flotte dans le milieu liquide du contenant C, entre deux eaux.

Dans cette configuration, l'organe indenteur 4 est soumis à des forces magnétiques par l'intermédiaire des aimants permanents 51, 52 des moyens magnétiques 5. Le courant I injecté dans les bobines électromagnétiques 54, 55 est à zéro.

Toujours dans cette configuration, l'organe indenteur 4 est stable selon les directions horizontale y et verticale z perpendiculaires à son axe longitudinal 4'.

En revanche, l'organe indenteur 4 est instable dans la direction horizontale instable x orientée coaxialement audit axe longitudinal 4'. La mesure de la force est alors destinée à être réalisée le long de cette direction horizontale instable x.

Pendant cette phase de préparation, les moyens magnétiques 5 génèrent un champ magnétique initial P1 qui permet le maintien de l'organe indenteur 4 dans une position initiale au repos, à distance de l'élément d'intérêt E.

Plus précisément, il s'exerce une force magnétique instable $\overline{F}_{mag}$ (orientée vers la gauche sur la figure 2), qui tend à écarter l'organe indenteur 4 par rapport à l'élément d'intérêt E.

L'extrémité distale 43 de l'organe indenteur 4 est alors maintenue au contact de la paroi latérale C2 du contenant C; l'extrémité proximale 42 de cet organe indenteur 4 se trouve à distance de l'élément d'intérêt E (par exemple quelques dizaines de micromètres).

Cette phase de préparation est suivie d'une phase de basculement de l'instabilité (l'extrémité proximale 42 vient au contact de l'élément d'intérêt E sans appliquer aucun effort sur ce dernier $\hat{F}_E$ = 0).

L'organe indenteur 4 perd le contact avec le contenant C lorsque le module de la force magnétique instable $\overline{F}_{mag}$ devient supérieur à la force d'adhésion entre l'organe indenteur 4 et le contenant C.

Les moyens de manœuvre 53 sont utilisés de manière à ajuster la position des aimants 51 et 52, et pour garantir $\hat{F}_E$ = 0 lorsque l'organe indenteur 4 est au contact de l'élément d'intérêt E juste avant le démarrage de la phase de chargement.

Le procédé est poursuivi par la phase de chargement au cours de laquelle l'organe indenteur 4 est manœuvrée en translation de sorte que son extrémité proximale 42 vienne générer une force de compression sur l'élément d'intérêt E porté par les moyens de maintien 3 (figure 2B).

A cet effet, les moyens magnétiques 5 sont pilotés de sorte à modifier le champ magnétique, depuis le champ magnétique initial P1 vers un champ magnétique modifié P2 (constant ou évolutif), dans lequel l'organe indenteur 4 est manœuvré en translation selon la direction horizontale instable x, cela dans un sens de chargement S1.

En l'espèce, la modification du champ magnétique, depuis le champ magnétique initial P1 vers le champ magnétique modifié P2, est obtenue par :

- une manœuvre en translation des deux aimants permanents 51, 52, selon la direction horizontale T et dans un premier sens T1 (vers la gauche sur la figure 2), et/ou
- en appliquant un courant I contrôlé et déterminé aux bobines électromagnétiques 54, 55.

Le cas échéant, la manœuvre en translation des deux aimants permanents 51, 52, selon la direction horizontale T, s'effectue sur la distance $x_{PI}$ contrôlée et déterminée.

En pratique, cette étape de chargement est avantageusement effectuée à une très faible vitesse de l'organe indenteur 4 (chargement quasi-statique), par exemple comprise entre 0,1 et 50 micromètres par seconde.

Le module de la force appliquée à l'élément d'intérêt E est alors avantageusement égal au module de la force magnétique instable $\vec{F}_{mag}$.

En pratique, la charge appliquée à l'élément d'intérêt E est ainsi modulée à partir du contrôle :

- de la distance $x_{PI}$ de manœuvre en translation des deux aimants permanents 51, 52, et/ou
- du courant I appliqué aux bobines électromagnétiques 54, 55.

Suite à cette phase de chargement, il est mis en œuvre une phase de déchargement au cours de laquelle l'extrémité proximale 42 de l'organe indenteur 4 est écartée par rapport à l'élément d'intérêt E qui est toujours maintenu par les moyens de maintien 3 (passage de la figure 2B vers la figure 2A).

Pour cela, les moyens magnétiques 5 sont pilotés de sorte à revenir au champ magnétique initial P1 dans lequel l'organe indenteur 4 est manœuvré en translation selon la direction horizontale instable x, cela dans un sens de déchargement S2.

En l'espèce, le retour au champ magnétique initial P1 est obtenu par :

- une manœuvre en translation (retour) des deux aimants permanents 51, 52 sur la distance $x_{PI}$, selon la direction horizontale T et dans un second sens T2 (inverse au premier sens T1), et/ou
- en annulant la valeur du courant I appliqué aux bobines électromagnétiques 54, 55.

Au cours de ce procédé, les moyens de détermination 7 effectuent une étape de collecte de la valeur D du déplacement en translation de l'organe indenteur 4 selon la direction horizontale instable x, et le cas échéant de la valeur du courant I appliqué aux bobines électromagnétiques 54, 55.

Cette étape de collecte est mise en œuvre au cours de la phase de chargement, et avantageusement aussi au cours de la phase de déchargement.

A partir des données collectées, les moyens de détermination 7 effectuent une étape de détermination des caractéristiques mécaniques de l'élément d'intérêt E, dite encore étape de visualisation de la réponse mécanique de l'élément d'intérêt E.

Comme précisé précédemment, cette étape de détermination (ou visualisation) tient avantageusement compte des caractéristiques du champ magnétique (notamment de la constante de raideur K et $K_{elec}$), de la valeur D du déplacement en translation de l'organe indenteur 4 selon la direction horizontale instable x et de la valeur du courant électrique I injecté dans les bobines électromagnétiques 54, 55.

Les données obtenues peuvent se présenter par exemple sous la forme d'une courbe caractéristique de la réponse mécanique de l'élément d'intérêt E mesurée lors d'un essai de charge / décharge à vitesse constante.

Cette courbe représente par exemple la corrélation entre, d'une part, la valeur de la force appliquée à l'élément d'intérêt et, d'autre part, la valeur de la distance de compression dudit élément d'intérêt par l'organe indenteur.

Une telle courbe est par exemple illustrée sur la figure 4 pour un élément d'intérêt E consistant en un ovocyte humain.

## Domaine d'application

Le dispositif de détermination 1 selon l'invention est ainsi adapté à la caractérisation mécanique d'un élément d'intérêt E.

Par « élément d'intérêt », on entend avantageusement un élément d'intérêt présentant une taille microscopique, c'est-à-dire un élément ayant une taille inférieure à 1 mm, de préférence entre $10\,\mu m$ et 1 mm.

Cet élément d'intérêt microscopique est avantageusement de nature biologique, c'est-à-dire par exemple une cellule, de préférence un ovocyte.

Une « cellule » est l'unité structurée constitutive de tout être vivant, formée d'un cytoplasme entouré d'une membrane et pouvant contenir un noyau.

Par « ovocyte », on entend la cellule sexuelle femelle des métazoaires, de préférence un ovocyte humain.

Dans ce cas, les composants utilisés, qui sont en contact avec l'ovocyte et son milieu de culture, sont avantageusement non-gamétotoxiques et à usage unique.

Ainsi, le dispositif de caractérisation mécanique 1 est tout-à-fait adapté à équiper un poste d'injection en procréation médicale assistée.

Un tel poste comporte classiquement une seringue de contention, une seringue d'injection et un microscope inversé.

Plus généralement, ce dispositif de détermination 1 selon l'invention peut être intéressant dans les domaines d'application où il est intéressant de mesurer des caractéristiques mécaniques sur tout type de cellules.

On peut citer les domaines suivants :

- le domaine de la reproduction, et des gamètes sens large (humain et animal) ;
- l'étude des cellules cancéreuses (la mesure des caractéristiques d'une cellule cancéreuse permet, entre autre, de mesurer si cette cellule est invasive) ;
- le domaine vétérinaire ;
- le domaine bactériologique ;
- le domaine de la zoologie et du monde unicellulaire ;
- le domaine de la botanique ;
- le domaine de la nanotribologie.

### Exemple

#### Matériel

Un exemple de dispositif de caractérisation 1 est décrit ci-dessous.

Les aimants permanents 51, 52 des moyens magnétiques 5 ont un diamètre et une longueur égale à 10 mm et une densité de charge égale à 1,25 A/m².

Ils sont disposés horizontalement, suivant l'axe x, de façon à respecter la configuration magnétique N-S N-S suivant leur axe longitudinal (axe x) et séparés d'une distance d égale à 10,4 cm.

L'organe indenteur 4, quant à lui, comporte les deux aimants permanents 45, de longueur égale à 1 mm et de diamètre égal à 0,5 mm, placés horizontalement. Leur magnétisation est également horizontale, orientée suivant l'axe x et dans le même sens. Elle vaut 3.10⁵ A/m.

La distance entre les deux aimants permanents 45 de l'organe indenteur 4 est égale à 1,5 cm.

Le centre de gravité G de l'organe indenteur 4 est défini au centre du segment séparant les aimants permanents 45.

La direction z représente la verticale de l'organe indenteur 4, direction pour laquelle le poids s'exerce.

#### Raideur du ressort magnétique

Pour effectuer des mesures sur les ovocytes, la gamme de force à appliquer est comprise entre 0 et environ 1μN.

Le débattement relatif à appliquer entre l'organe indenteur 4 et les aimants permanents externes 51, 52 se situera dans la fourchette [-2 mm, 2 mm].

En effet si $G^x$ = 2 mm alors $F_{mag}^x$ = 2,6 μN.

Un tel effort permet d'assurer un chargement sur l'ensemble des ovocytes humains dans le cadre de l'assistance médicale à la procréation.

Une courbe, pour laquelle une fonction affine a été superposée sur l'intervalle [-2 mm, 2 mm], permet de valider la linéarité de $F_{mag}^x$ en fonction de $G^x$:

$$F_{mag}^x = K. G^x$$

avec K la raideur du ressort magnétique suivant la direction x.

Dans la configuration retenue, la raideur du ressort magnétique vaut 0,0013 N/m. Il s'agit d'une valeur très faible par rapport, par exemple, à la raideur du levier d'un microscope à force atomique dont les leviers les plus souples sont environ dix fois plus raides.

#### Résultat

La figure 4 représente la réponse mécanique d'un ovocyte immature.

L'ovocyte présente un diamètre égal à 144 μm et un diamètre de cytoplasme de 106 μm.

La courbe d'évolution de la force $\hat{F}_{oo}$ illustre la phase de chargement (partie supérieure - V1).

Au cours de cette phase, l'ovocyte est comprimé par l'organe indenteur jusqu'à atteindre une distance de compression $d_{oo}^{max}$ égale à 14 μm qui correspond à la déformation maximale de l'ovocyte pour une force $\hat{F}_{oo}^{max}$ de 150 nN (repère carré Z1 sur la figure 4).

La partie inférieure de la courbe (V2) représente la phase de décharge. Elle correspond au recul de l'organe indenteur jusqu'au repère carré Z2 de la figure 4.

Cette courbe met bien en évidence une hystérésis, due à la nature visqueuse de l'ovocyte. Une déformation résiduelle ($d_r$) de l'ovocyte est observable à la fin du déchargement.

### Revendications

1. Dispositif pour la caractérisation mécanique d'un élément d'intérêt (E), avantageusement un élément d'intérêt microscopique, éventuellement de nature biologique, voire une cellule,
lequel dispositif de caractérisation mécanique (1) comprend :

   - des moyens supports (2), pour la réception d'un contenant (C) adapté à contenir un milieu liquide,
   - des moyens de maintien (3), pour le maintien dudit élément d'intérêt (E) dans ledit milieu liquide, le dispositif de caractérisation mécanique est **caractérisé en ce qu'**il comprend:

- un organe indenteur (4) présentant un axe longitudinal (4') et destiné à rester en sustentation dans ledit milieu liquide avec ledit axe longitudinal (4') orienté horizontalement, lequel organe indenteur (4) comporte au moins un aimant permanent (45) et une extrémité proximale (42) destinée à indenter ledit élément d'intérêt (E),
- des moyens magnétiques (5), pour générer un champ magnétique dans lequel ledit organe indenteur (4) est destiné à se déplacer et dans lequel ledit organe indenteur (4) est instable dans une direction horizontale instable (x) orientée coaxialement audit axe longitudinal (4'), et qui participe à la sustentation dudit organe indenteur (4) avec ladite direction horizontale instable (x),
- des moyens de commande (6), destinés à piloter lesdits moyens magnétiques (5) de sorte à générer une variation dudit champ magnétique qui est adaptée à manœuvrer ledit organe indenteur (4) en translation selon ladite direction horizontale instable (x), et
- des moyens (7) pour déterminer les caractéristiques mécaniques dudit élément d'intérêt (E), cela tenant compte des caractéristiques dudit champ magnétique et de la valeur (D) du déplacement en translation dudit organe indenteur (4) selon ladite direction horizontale instable (x) lorsque ladite extrémité proximale (42) dudit organe indenteur (4) génère une force de compression sur ledit élément d'intérêt (E) porté par lesdits moyens de maintien (3).

2. Dispositif pour la caractérisation mécanique d'un élément d'intérêt (E), selon la revendication 1, **caractérisé en ce que** l'organe indenteur (4) comporte au moins deux aimants permanents (45) qui sont agencés :

- avec des champs magnétiques coaxiaux et dans un même sens NS - NS, et
- dans une position d'équilibrage horizontal, dans laquelle le centre de poussée est destiné à être confondu avec le centre de gravité.

3. Dispositif pour la caractérisation mécanique d'un élément d'intérêt (E), selon l'une quelconque des revendications 1 ou 2, **caractérisé en ce que** ledit organe indenteur (4) comprend un corps (41) réalisé par un capillaire délimitant une chambre étanche (44) qui est remplie d'air et qui renferme ledit au moins un aimant permanent (45).

4. Dispositif pour la caractérisation mécanique d'un élément d'intérêt (E), selon l'une quelconque des revendications 1 à 3, **caractérisé en ce que** le contenant (C) comprend un fond (C1) raccordé à une paroi latérale (C2) dont une bordure supérieure libre (C21) délimite une ouverture supérieure (C22), par exemple une boîte de Pétri.

5. Dispositif pour la caractérisation mécanique d'un élément d'intérêt (E), selon l'une quelconque des revendications 1 à 4, **caractérisé en ce que** les moyens magnétiques (5) comprennent :

- au moins deux aimants permanents (51, 52) qui sont agencés coaxialement, selon un axe horizontal (A), et dans le même sens NS - NS, et
- des moyens (53) pour la manœuvre en translation desdits aimants permanents (51, 52).

6. Dispositif pour la caractérisation mécanique d'un élément d'intérêt (E), selon la revendication 5, **caractérisé en ce que** lesdits au moins deux aimants permanents (51, 52) sont disposés chacun au sein d'une bobine électromagnétique (54, 55), lesquelles bobines électromagnétiques (54, 55) sont agencées coaxialement selon l'axe horizontal (A) et sont raccordées à des moyens (56) pour piloter le courant électrique alimentant lesdites bobines électromagnétiques (54, 55).

7. Dispositif pour la caractérisation mécanique d'un élément d'intérêt (E), selon l'une quelconque des revendications 5 ou 6, **caractérisé en ce que** lesdits au moins deux aimants permanents (51, 52), et le cas échéant lesdites au moins deux bobines électromagnétiques (54, 55), sont disposés de part et d'autre du contenant (C), à une distance constante d l'un par rapport à l'autre.

8. Dispositif pour la caractérisation mécanique d'un élément d'intérêt (E), selon l'une quelconque des revendications 1 à 7, **caractérisé en ce que** les moyens de maintien (3) comprennent une pipette d'aspiration (31).

9. Dispositif pour la caractérisation mécanique d'un élément d'intérêt (E), selon l'une quelconque des revendications 1 à 8, **caractérisé en ce que** les moyens (7) pour déterminer les caractéristiques mécaniques dudit élément d'intérêt (E) comprennent des moyens (71, 72) pour déterminer la valeur (D) de déplacement en translation de l'organe indenteur (4) selon ladite direction horizontale instable (x).

10. Poste d'injection en procréation médicale assistée, équipé d'un dispositif selon l'une quelconque des revendications 1 à 9.

11. Procédé pour étudier les caractéristiques mécaniques d'un élément d'intérêt (E), avantageusement un élément d'intérêt (E) microscopique, éventuellement de nature biologique, voire une cellule, par la mise en œuvre d'un dispositif (1) selon l'une quelconque des revendications 1 à 9,
lequel procédé comprend :

a) une phase de préparation au cours de laquelle, d'une part, ledit élément d'intérêt (E) est maintenu dans le milieu liquide du contenant (C) par lesdits moyens de maintien (3) et, d'autre part, les moyens magnétiques (5) génèrent un champ magnétique initial (P1) qui permet le maintien dudit organe indenteur (4) dans une position initiale au repos, à distance dudit élément d'intérêt (E),

b) une phase de chargement au cours de laquelle les moyens magnétiques (5) sont pilotés de sorte à modifier le champ magnétique, depuis ledit champ magnétique initial (P1) jusqu'à un champ magnétique modifié (P2), dans lequel ledit organe indenteur (4) est manœuvré en translation selon ladite direction horizontale instable (x), cela dans un sens de chargement dans lequel ladite extrémité proximale (42) dudit organe indenteur (4) génère une force de compression sur ledit élément d'intérêt (E) maintenu par lesdits moyens de maintien (3),

c) une phase de déchargement au cours de laquelle les moyens magnétiques (5) sont pilotés de sorte à rétablir ledit champ magnétique initial (P1) dans lequel ledit organe indenteur (4) est manœuvré en translation selon ladite direction horizontale instable (x), cela dans un sens de déchargement dans lequel ladite extrémité proximale (42) dudit organe indenteur (4) s'écarte par rapport audit élément d'intérêt (E) maintenu par lesdits moyens de maintien (3),

lequel procédé comprend une étape de collecte de la valeur (D) du déplacement en translation dudit organe indenteur (4) selon ladite direction horizontale instable (x), au moins au cours de la phase de chargement, et

lequel procédé comprend une étape de détermination des caractéristiques mécaniques dudit élément d'intérêt (E), tenant compte des caractéristiques dudit champ magnétique et de la valeur (D) du déplacement en translation dudit organe indenteur (4) selon ladite direction horizontale instable (x).

12. Procédé selon la revendication 11, en combinaison avec les revendications 5 ou 6, **caractérisé en ce que** les caractéristiques dudit champ magnétique sont fonction de :

- la valeur de déplacement des moyens magnétiques (5) et/ou
- la valeur du courant électrique injecté dans les bobines électromagnétiques (54, 55).

13. Procédé selon l'une quelconque des revendications 11 ou 12, **caractérisé en ce que** l'élément d'intérêt (E) est une cellule choisie parmi :

- les ovocytes, ou
- les cellules cancéreuses.

**Patentansprüche**

1. Vorrichtung zur mechanischen Charakterisierung eines Elements von Interesse (E), vorteilhafterweise eines mikroskopischen Elements von Interesse, gegebenenfalls biologischer Art, oder sogar einer Zelle, wobei die Vorrichtung zur mechanischen Charakterisierung (1) umfasst:

- Trägermittel (2) zum Aufnehmen eines Behältnisses (C), das geeignet ist, ein flüssiges Medium zu enthalten,
- Haltemittel (3) zum Halten des Elements von Interesse (E) in dem flüssigen Medium, wobei die Vorrichtung zur mechanischen Charakterisierung **dadurch gekennzeichnet ist, dass** sie umfasst:
- einen Eindringkörper (4), der eine Längsachse (4') aufweist und dazu bestimmt ist, in dem flüssigen Medium mit der horizontal ausgerichteten Längsachse (4') in Schwebe zu bleiben, wobei der Eindringkörper (4) mindestens einen Permanentmagneten (45) und ein proximales Ende (42), das dazu bestimmt ist, in das Element von Interesse (E) gedrückt zu werden, beinhaltet,
- magnetische Mittel (5), um ein Magnetfeld zu erzeugen, in dem sich der Eindringkörper (4) verlagern soll und in dem der Eindringkörper (4) in einer koaxial zu der Längsachse (4') ausgerichteten instabilen horizontalen Richtung (x) instabil ist und das zum Schweben des Eindringkörpers (4) mit der instabilen horizontalen Richtung (x) beiträgt,
- Steuermittel (6), die dazu bestimmt sind, die magnetischen Mittel (5) so anzusteuern, dass eine Änderung des Magnetfelds erzeugt wird, die geeignet ist, den Eindringkörper (4) translatorisch entlang der instabilen horizontalen Richtung (x) zu betätigen, und
- Mittel (7) zum Bestimmen der mechanischen Merkmale des relevanten Elements (E), und dies unter Berücksichtigung der Merkmale des Magnetfelds und des Werts (D) der translatorischen Verlagerung des Eindringkörpers (4) entlang der instabilen horizontalen Achse (x), wenn das proximale Ende (42) des Eindringkörpers (4) eine Druckkraft auf das von den Haltemitteln (3) getragene Element von Interesse (E) erzeugt.

2. Vorrichtung zur mechanischen Charakterisierung eines Elements von Interesse (E) nach Anspruch 1, **dadurch gekennzeichnet, dass** der Eindringkörper (4) mindestens zwei Permanentmagnete (45) beinhaltet, die angeordnet sind:

    - mit koaxialen Magnetfeldern und gleichsinnig NS - NS und
    - in einer horizontalen Gleichgewichtsposition, in welcher der Angriffspunkt dazu bestimmt ist, mit dem Schwerpunkt zusammenzufallen.

3. Vorrichtung zur mechanischen Charakterisierung eines Elements von Interesse (E) nach einem der Ansprüche 1 oder 2, **dadurch gekennzeichnet, dass** der Eindringkörper (4) ein Gehäuse (41) umfasst, das durch eine Kapillare ausgeführt ist, die eine dichte Kammer (44) begrenzt, die mit Luft gefüllt ist und die den mindestens einen Permanentmagneten (45) einschließt.

4. Vorrichtung zur mechanischen Charakterisierung eines Elements von Interesse (E) nach einem der Ansprüche 1 bis 3, **dadurch gekennzeichnet, dass** das Behältnis (C) einen Boden (C1) umfasst, der mit einer Seitenwand (C2) verbunden ist, deren eine freie obere Kante (C21) eine obere Öffnung (C22) begrenzt, beispielsweise eine Petrischale.

5. Vorrichtung zur mechanischen Charakterisierung eines Elements von Interesse (E) nach einem der Ansprüche 1 bis 4, **dadurch gekennzeichnet, dass** die magnetischen Mittel (5) umfassen:

    - mindestens zwei Permanentmagnete (51, 52), die koaxial angeordnet sind, entlang einer horizontalen Achse (A) und gleichsinnig NS - NS, und
    - Mittel (53) zur translatorischen Betätigung der Permanentmagnete (51, 52).

6. Vorrichtung zur mechanischen Charakterisierung eines Elements von Interesse (E) nach Anspruch 5, **dadurch gekennzeichnet, dass** die mindestens zwei Permanentmagnete (51, 52) jeweils im Inneren einer Elektromagnetspule (54, 55) angeordnet sind, wobei die Elektromagnetspulen (54, 55) koaxial entlang der horizontalen Achse (A) angeordnet sind und mit Mitteln (56) zum Ansteuern des die Elektromagnetspulen (54, 55) versorgenden elektrischen Stroms verbunden sind.

7. Vorrichtung zur mechanischen Charakterisierung eines Elements von Interesse (E) nach einem der Ansprüche 5 oder 6, **dadurch gekennzeichnet, dass** die mindestens zwei Permanentmagnete (51, 52) und gegebenenfalls die mindestens zwei Elektromagnetspulen (54, 55) beidseits des Behältnisses (C) angeordnet sind, in einem konstanten Abstand d zueinander.

8. Vorrichtung zur mechanischen Charakterisierung eines Elements von Interesse (E) nach einem der Ansprüche 1 bis 7, **dadurch gekennzeichnet, dass** die Haltemittel (3) eine Ansaugpipette (31) umfassen.

9. Vorrichtung zur mechanischen Charakterisierung eines Elements von Interesse (E) nach einem der Ansprüche 1 bis 8, **dadurch gekennzeichnet, dass** die Mittel (7) zum Bestimmen der mechanischen Merkmale des Elements von Interesse (E) Mittel (71, 72) zum Bestimmen des Werts (D) der translatorischen Verlagerung des Eindringkörpers (4) entlang der instabilen horizontalen Richtung (x) umfassen.

10. Injektionsstation zur medizinisch unterstützten Fortpflanzung, die mit einer Vorrichtung nach einem der Ansprüche 1 bis 9 ausgestattet ist.

11. Verfahren zur Untersuchung der mechanischen Merkmale eines Elements von Interesse (E), vorteilhafterweise eines mikroskopischen Elements von Interesse (E), gegebenenfalls biologischer Art, oder sogar einer Zelle, durch den Einsatz einer Vorrichtung (1) nach einem der Ansprüche 1 bis 9, wobei das Verfahren umfasst:

    a) eine Vorbereitungsphase, während der zum einen das Element von Interesse (E) in dem flüssigen Medium des Behältnisses (C) durch die Haltemittel (3) gehalten wird und zum anderen die magnetischen Mittel (5) ein Ausgangsmagnetfeld (P1) erzeugen, welches das Halten des Eindringkörpers (4) in einer Ausgangsposition in Ruhe, vom Element von Interesse (E) beabstandet, ermöglicht,
    b) eine Belastungsphase, während der die magnetischen Mittel (5) so angesteuert werden, dass das Magnetfeld geändert wird, von dem Ausgangsmagnetfeld (P1) bis zu einem geänderten Magnetfeld (P2), wobei der Eindringkörper (4) translatorisch entlang der instabilen horizontalen Richtung (x) betätigt wird, und dies in eine Belastungsrichtung, in der das proximale Ende (42) des Eindringkörpers (4) eine Druckkraft auf das von den Haltemitteln (3) gehaltene Element von Interesse (E) erzeugt,
    c) eine Entlastungsphase, während der die magnetischen Mittel (5) so angesteuert werden, dass das Ausgangsmagnetfeld (P1) wiederhergestellt wird, wobei der Eindringkörper (4) translatorisch entlang der instabilen horizontalen Richtung (x) betätigt wird, und dies in eine Entlastungsrichtung, in der sich das proximale Ende (42) des Eindringkörpers (4) von dem von den Haltemitteln (3) gehaltenen Element von Interesse (E) entfernt,

wobei das Verfahren einen Schritt des Erfassens des Werts (D) der translatorischen Verlagerung des Eindringkörpers (4) entlang der instabilen horizontalen Richtung (x) umfasst, mindestens während der Belastungsphase, und wobei das Verfahren einen Schritt des Bestimmens der mechanischen Merkmale des Elements von Interesse (E) umfasst, unter Berücksichtigung der Merkmale des Magnetfelds und des Werts (D) der translatorischen Verlagerung des Eindringkörpers (4) entlang der instabilen horizontalen Richtung (x).

**12.** Verfahren nach Anspruch 11 in Kombination mit den Ansprüchen 5 oder 6, **dadurch gekennzeichnet, dass** die Merkmale des Magnetfelds abhängig sind von:

     - dem Wert der Verlagerung der magnetischen Mittel (5) und/oder
     - dem Wert des elektrischen Stroms, der in die Elektromagnetspulen (54, 55) eingeleitet wird.

**13.** Verfahren nach einem der Ansprüche 11 oder 12, **dadurch gekennzeichnet, dass** das Element von Interesse (E) eine Zelle ist, die ausgewählt ist unter:

     - Oozyten oder
     - Krebszellen.

**Claims**

**1.** A device for mechanically characterizing an element of interest (E), advantageously a microscopic element of interest, possibly biological in nature, or even a cell,

     wherein the mechanical characterization device (1) comprises:

         - support means (2), for receiving a container (C) suitable for containing a liquid medium,
         - holding means (3), for holding said element of interest (E) in said liquid medium,

     the device for mechanically characterizing is **characterized in that** it comprises:

         - an indenting member (4) having a longitudinal axis (4') and intended to remain in suspension in said liquid medium with said longitudinal axis (4') oriented horizontally, wherein said indenting member (4) includes at least one permanent magnet (45) and one proximal end (42) intended to indent said element of interest (E),
- magnetic means (5), for generating a magnetic field in which said indenting member (4) is intended to move and in which said indenting member (4) is unstable in an unstable horizontal direction (x) oriented coaxially to said longitudinal axis (4') and which participates in the suspension of said indenting member (4) with said unstable horizontal direction (x),
- control means (6), intended to pilot said magnetic means (5) so as to generate a variation of said magnetic field that is adapted to operate said indenting member (4) in translation along said unstable horizontal direction (x), and
- means (7) for determining the mechanical characteristics of said element of interest (E), taking into account the characteristics of said magnetic field and the value (D) of translational displacement of said indenting member (4) along said unstable horizontal direction (x) when said proximal end (42) of said indenting member (4) generates a compression force to said element of interest (E).

**2.** The device for mechanically characterizing an element of interest (E) according to claim 1, **characterized in that** the indenting member (4) includes at least two permanent magnets (45) that are arranged:

     - with magnetic fields coaxial to each other and in a same direction NS - NS, and
     - in a horizontal equilibrium position, in which the centre of buoyancy is intended to be merged with the centre of gravity.

**3.** The device for mechanically characterizing an element of interest (E) according to any one of claims 1 or 2, **characterized in that** said indenting member (4) comprises a body (41) consisted of a capillary delimiting a sealed chamber (44) that is filled with air and that contains said at least one permanent magnet (45).

**4.** The device for mechanically characterizing an element of interest (E) according to any one of claims 1 to 3, **characterized in that** the container (C) comprises a bottom (C1) connected to a lateral wall (C2), a free upper edge (C21) of which delimits an upper opening (C22), for example a Petri dish.

**5.** The device for mechanically characterizing an element of interest (E) according to any one of claims 1 to 4, **characterized in that** the magnetic means (5) comprise:

- at least two permanent magnets (51, 52) that are arranged coaxially, along a horizontal axis (A), and in the same direction NS - NS, and
- means (53) for operating said permanent magnets (51, 52) in translation.

6. The device for mechanically characterizing an element of interest (E) according to claim 5, **characterized in that** said at least two permanent magnets (51, 52) are each arranged within an electromagnetic coil (54, 55),
wherein said electromagnetic coils (54, 55) are arranged coaxially along the horizontal axis (A) and are connected to means (56) for piloting the electric current powering said electromagnetic coils (54, 55).

7. The device for mechanically characterizing an element of interest (E) according to any one of claims 5 or 6, **characterized in that** said at least two permanent magnets (51, 52), and, as the case may be, said at least two electromagnetic coils (54, 55), are arranged on either side of the container (C), at a constant distance d from each other.

8. The device for mechanically characterizing an element of interest (E) according to any one of claims 1 to 7, **characterized in that** the holding means (3) comprise a suction pipette (31).

9. The device for mechanically characterizing an element of interest (E) according to any one of claims 1 to 8, **characterized in that** the means (7) for determining the mechanical characteristics of said element of interest (E) comprise means (71, 72) for determining the value (D) of translational displacement of the indenting member (4) along said unstable horizontal direction (x).

10. A medically assisted procreation injection station, equipped with a device according to any one of claims 1 to 9.

11. A method for studying the mechanical characteristics of an element of interest (E), advantageously a microscopic element of interest (E), possibly biological in nature, or even a cell, by implementing a device (1) according to any one of claims 1 to 9,

wherein the method comprises:

a) a preparation phase in which, on the one hand, said element of interest (E) is held in the liquid medium of the container (C) by said holding means (3) and, on the other hand, the magnetic means (5) generate an initial magnetic field (P1) that allows holding said indenting member (4) in an initial position at rest, remote from said element of interest (E),
b) a loading phase during which the magnetic means (5) are piloted so as to modify the magnetic field from said initial magnetic field (P1) to a modified magnetic field (P2), wherein said indenting member (4) is operated in translation along said unstable horizontal direction (x), in a loading direction in which said proximal end (42) of said indenting member (4) generates a compression force to said element of interest (E) held by said holding means (3),
c) an unloading phase during which the magnetic means (5) are piloted so as to restore said initial magnetic field (P1) in which said indenting member (4) is operated in translation along said unstable horizontal direction (x), in an unloading direction in which said proximal end (42) of said indenting member (4) moves apart from said element of interest (E) held by said holding means (3),

said method comprises a step of collecting the value (D) of translational displacement of said indenting member (4) along said unstable horizontal direction (x), at least during the loading phase, and
said method comprises a step of determining mechanical characteristics of said element of interest (E), taking into account the characteristics of said magnetic field (E) and the value (D) of translational displacement of said indenting member (4) along said unstable horizontal direction (x).

12. The method according to claim 11, in combination with claims 5 or 6. **characterized in that** the characteristics of said magnetic field are function of:

- the value of displacement of the magnetic means (5) and/or
- the value of the electric current injected into the electromagnetic coils (54, 55).

13. The method according to any one of claims 11 or 12, **characterized in that** the element of interest (E) is a cell chosen among:

- the oocytes, or
- the cancer cell.

# Fig.1

# Fig.2

# Fig.3

# Fig.4

**EP 3 602 000 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008105919 A

- US 2011053241 A

**Littérature non-brevet citée dans la description**

- **GOUILLOU et al.** *SCIENTIFIC REPORTS,* 09 Février 2016, vol. 6